# EUROPÄISCHE PATENTANMELDUNG

(11) **EP 4 670 638 A1**
(43) Veröffentlichungstag der Anmeldung: **31.12.2025**
(21) Anmeldenummer: 24185400.9
(22) Anmeldetag: 28.06.2024
(51) Int. Cl.: A61B 6/04

(54) **VERFAHREN UND SYSTEM ZUM EINSTELLEN EINER POSITION EINES UNTERSUCHUNGSOBJEKTS**

(71) Anmelder: Siemens Healthineers AG, 91301 Forchheim (DE)
(72) Erfinder: Ditt, Hendrik, 91413 Neustadt an der Aisch (DE); Taubmann, Oliver, 91365 Weilersbach (DE); Wimmer, Andreas, 91301 Forchheim (DE)
(74) Vertreter: Siemens Healthineers Patent Attorneys

(57) **Zusammenfassung**

Die Erfindung betrifft ein Verfahren zum Einstellen einer Position eines Untersuchungsobjekts relativ zu einer Gantry eines medizinischen Bildgebungsgeräts, das Verfahren umfassend:
- ein Lagern des Untersuchungsobjekts auf einer Lagerungsstruktur eines Untersuchungstisches des medizinischen Bildgebungsgeräts,
- ein Erfassen von Bilddaten, welche das Untersuchungsobjekt betreffen,
- ein Berechnen einer Ist-Stellung, welche das Untersuchungsobjekt betrifft, basierend auf den Bilddaten,
- ein Berechnen, basierend auf der Ist-Stellung und einer Soll-Stellung, welche das Untersuchungsobjekt betrifft, von Einstelldaten für das Einstellsystem, um die Ist-Stellung durch die laterale Translationsbewegung der Lagerungsstruktur relativ zu der Systemachse im Sinne einer Angleichung an die Soll-Stellung zu beeinflussen, wobei die laterale Translationsbewegung horizontal und zu der Systemachse senkrecht ist,
- ein Einstellen der Position des Untersuchungsobjekts relativ zu der Gantry, indem die laterale Translationsbewegung der Lagerungsstruktur relativ zu der Systemachse mittels des Einstellsystems basierend auf den Einstelldaten ausgeführt wird.

## Beschreibung

Die Erfindung betrifft ein Verfahren zum Einstellen einer Position eines Untersuchungsobjekts relativ zu einer Gantry. Die Erfindung betrifft ferner ein Verfahren zum Bereitstellen von Bildgebungsdaten und ein System.

Insbesondere bei einer Kopf-Bildgebung ist die Ausrichtung des Untersuchungsobjekts relativ zu der Gantry des medizinischen Bildgebungsgeräts wichtig, um eine Verfälschung bei den rekonstruierten Bildwerten zu vermeiden. Gerade bei der Kopf-Bildgebung sind feine HU-Unterschiede von großer Bedeutung, sodass beispielsweise ein Unterschied von 1 HU schon zu einer anderen Diagnose führen kann. Durch nicht-zentrische Lagerung können sich Bildwerte je nach Position allerdings um mehrere HU ändern.

Neben der Höhe des Tisches, die beispielsweise automatisch angepasst werden kann, ist für eine gute Schädelaufnahme daher auch eine laterale Ausrichtung des Schädels zu berücksichtigen. Diese kann beispielsweise manuell durch das medizinische Personal mit Hilfe von Kissen oder Ähnlichem erreicht werden. Jedoch besteht das Risiko, dass sich in der Zeit zwischen dieser Ausrichtung und dem Beginn der Akquisition der Bildgebungsdaten die Position des Untersuchungsobjekts relativ zu der Gantry verändert. Insbesondere dann, wenn das Untersuchungsobjekt ein Körperteil eines Menschen oder Tieres ist, kann eine Positionsänderung des Untersuchungsobjekts relativ zu der Gantry aufgrund von Neugier oder mangelndem Komfort erfolgen.

Als Stand der Technik ist hierbei die DE 10 2020 213 690 A1 zu nennen.

Die Erfindung hat die Aufgabe, eine Alternative zu herkömmlichen Lösungen für das Einstellen einer Position eines Untersuchungsobjekts relativ zu einer Gantry, insbesondere in Bezug auf eine laterale Ausrichtung, bereitzustellen. Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft ein Verfahren zum Einstellen einer Position eines Untersuchungsobjekts relativ zu einer Gantry eines medizinischen Bildgebungsgeräts, das Verfahren umfassend:
- ein Lagern des Untersuchungsobjekts auf einer Lagerungsstruktur eines Untersuchungstisches des medizinischen Bildgebungsgeräts, wobei das medizinische Bildgebungsgerät die Gantry mit einer Öffnung aufweist, wobei sich die Öffnung derart tunnelförmig entlang einer Systemachse der Gantry erstreckt, dass die Lagerungsstruktur entlang der Systemachse in die Öffnung einführbar ist, wobei der Untersuchungstisch ein Einstellsystem aufweist, wobei mittels des Einstellsystems eine laterale Translationsbewegung der Lagerungsstruktur relativ zu der Systemachse ausgeführt werden kann, wobei die laterale Translationsbewegung horizontal und zu der Systemachse senkrecht ist,
- ein Erfassen von Bilddaten, welche das Untersuchungsobjekt betreffen,
- ein Berechnen einer Ist-Stellung, welche das Untersuchungsobjekt betrifft, basierend auf den Bilddaten,
- ein Berechnen, basierend auf der Ist-Stellung und einer Soll-Stellung, welche das Untersuchungsobjekt betrifft, von Einstelldaten für das Einstellsystem, um die Ist-Stellung durch die laterale Translationsbewegung der Lagerungsstruktur relativ zu der Systemachse im Sinne einer Angleichung an die Soll-Stellung zu beeinflussen,
- ein Einstellen der Position des Untersuchungsobjekts relativ zu der Gantry, indem die laterale Translationsbewegung der Lagerungsstruktur relativ zu der Systemachse mittels des Einstellsystems basierend auf den Einstelldaten ausgeführt wird.

Das Verfahren zum Einstellen einer Position eines Untersuchungsobjekts relativ zu einer Gantry kann insbesondere computerimplementiert sein. Die Systemachse kann insbesondere horizontal sein. Das Untersuchungsobjekt kann beispielsweise ein Körperteil, insbesondere ein Kopf, eines Menschen oder eines Tieres oder ein Phantom sein. Die Ist-Stellung kann beispielsweise aufgrund einer Positionsänderung des Untersuchungsobjekts relativ zu der Lagerungsstruktur von der Soll-Stellung abweichen. Die Positionsänderung des Untersuchungsobjekts relativ zu der Lagerungsstruktur kann beispielsweise horizontal und zu der Systemachse senkrecht sein und/oder nach einem initialen Positionieren des Untersuchungsobjekts in einer Soll-Stellung erfolgen. Die Ist-Stellung kann beispielsweise aufgrund einer Positionsänderung des Untersuchungstisches relativ zu der Gantry von der Soll-Stellung abweichen. Die Positionsänderung des Untersuchungstisches relativ zu der Gantry kann beispielsweise horizontal und zu der Systemachse senkrecht sein und/oder nach einem initialen Positionieren des Untersuchungsobjekts in einer Soll-Stellung erfolgen, beispielsweise aufgrund einer Kollision, ins-besondere aufgrund einer Kollision mit sich im Untersuchungsraum bewegenden Personen und/oder Geräten, erfolgen.

Die korrekte Ausrichtung des Untersuchungsobjekts kann somit automatisch überprüft werden. Eine Korrektur der Abweichung kann durch das medizinische Personal nach entsprechender Anleitung oder automatisch vorgenommen werden. Insbesondere wenn es sich um eine laterale Verschiebung von bis zu 3 cm handelt, kann diese auch automatisch vorgenommen werden. Damit wird ein zuverlässigeres Ergebnis der Bildgebungsuntersuchung ermöglicht.

Eine Ausführungsform sieht vor, dass ein initialer Bilddatensatz, welcher das Untersuchungsobjekt betrifft, empfangen wird, wobei die Soll-Stellung basierend auf dem initialen Bilddatensatz berechnet wird. Der initiale Bilddatensatz kann beispielsweise radiologisch sein und/oder mittels des medizinischen Bildgebungsgeräts erfasst werden. Der initiale Bilddatensatz kann beispielsweise optisch sein und/oder mittels einer Kamera erfasst werden. Der initiale Bilddatensatz kann insbesondere eine Momentaufnahme des Untersuchungsobjekts in der Soll-Stellung sein und/oder unmittelbar nach dem initialen Positionieren erfasst werden. Der initiale Bilddatensatz kann beispielsweise auf einer Scout-Bildaufnahme und/oder auf einer Localizer-Bildaufnahme basieren.

Eine Ausführungsform sieht vor, dass eine Soll-Pose des Untersuchungsobjekts basierend auf dem initialen Bilddatensatz berechnet wird, wobei die Soll-Stellung basierend auf der Soll-Pose des Untersuchungsobjekts berechnet wird.

Bei dem Berechnen der Soll-Pose kann beispielsweise basierend auf den radiologischen Bilddaten eine Orbito-Meatal-Linie beispielsweise mittels künstlicher Intelligenz und/oder unter Zuhilfenahme von a priori Wissen automatisiert erkannt werden und/oder ein optimaler Inklinationswinkel des Untersuchungsobjekts berechnet werden. Die Soll-Pose kann insbesondere einer regelkonformen Lagerung des Untersuchungsobjekts entsprechen. Die Soll-Pose des Untersuchungsobjekts kann insbesondere eine Soll-Position des Untersuchungsobjekts und eine Soll-Orientierung des Untersuchungsobjekts betreffen.

Insbesondere kann basierend auf den radiologischen Bilddaten ein erstes virtuelles dreidimensionales Modell, beispielsweise in Form eines Soll-Avatars, für das Untersuchungsobjekt generiert werden. Insbesondere kann der Satz von Landmarken basierend auf dem ersten virtuellen dreidimensionalen Modell berechnet werden. Ferner kann ein Zielbild berechnet werden, welches das erste virtuelle dreidimensionale Modell in der Soll-Pose des Untersuchungsobjekts zeigt. Insbesondere kann jede Landmarke des Satzes von Landmarken anatomisch sein und/oder optisch sichtbar sein. Der Satz von Landmarken kann insbesondere ferner basierend auf den optischen Bilddaten berechnet werden.

Die Soll-Stellung kann insbesondere derart berechnet werden, dass das Untersuchungsobjekt in der Soll-Stellung gelagert ist, wenn der Satz von Landmarken in der Soll-Stellung angeordnet ist, insbesondere wenn die Ist-Stellung der Soll-Pose gleicht. Die Soll-Stellung kann beispielsweise basierend auf einer Anwendung von künstlicher Intelligenz und/oder a priori-Wissen auf die radiologischen Bilddaten und die Soll-Pose berechnet werden. Die Soll-Stellung der Landmarken kann in das Zielbild projiziert werden.

Insbesondere kann basierend auf den optischen Bilddaten ein zweites virtuelles dreidimensionales Modell, beispielsweise in Form eines Ist-Avatars, für das Untersuchungsobjekt generiert werden. Der Satz von Landmarken kann beispielsweise basierend auf dem zweiten virtuellen dreidimensionalen Modell erkannt werden. Insbesondere kann die Ist-Stellung basierend auf dem zweiten virtuellen dreidimensionalen Modell und/oder basierend auf einer Anwendung von künstlicher Intelligenz und/oder a priori-Wissen auf die optischen Bilddaten berechnet werden.

Bei dem Berechnen der Einstelldaten für das Einstellsystem kann beispielsweise berechnet werden, wie sich die Position der Lagerungsstruktur relativ zu der Systemachse verändern muss, damit eine Angleichung der Ist-Stellung an die Soll-Stellung erfolgen kann. Eine Angleichung der Ist-Stellung an die Soll-Stellung kann insbesondere erfolgen, indem ein Unterschied zwischen der Ist-Stellung und der Soll-Stellung minimiert wird.

Das Berechnen der Einstelldaten für das Einstellsystem kann beispielsweise basierend auf einem Maschinenlernalgorithmus erfolgen. Unter einem Maschinenlernalgorithmus wird im Kontext dieser Anmeldung insbesondere ein Algorithmus, der zum Maschinellen Lernen ausgebildet ist, verstanden. Der Maschinenlernalgorithmus kann beispielsweise zum überwachten Lernen und/oder zum unüberwachten Lernen ausgebildet sein. Der Maschinenlernalgorithmus kann beispielsweise zum tiefen Lernen ("deep learning") und/oder zum bestärkendem Lernen ("reinforcement learning") und/oder zum Marginal Space Learning ausgebildet sein.

Der Maschinenlernalgorithmus kann beispielsweise auf Entscheidungsbäumen ("decision trees"), einem Random Forest, einer logistischen Regression, einer Support Vector Machine, einem künstlichen neuronalen Netzwerk, insbesondere einem konvolutionellen neuronalen Netzwerk ("convolutional neural network") und/oder einem rekurrenten neuronalen Netzwerk ("recurrent neural network"), einer Kernel-Methode, Bayes-Klassifikatoren oder ähnlichem oder auf Kombinationen davon basieren.

Eine Ausführungsform sieht vor, dass die Ist-Stellung eine Landmarken-Ist-Stellung, welche einen Satz von Landmarken an dem Untersuchungsobjekt betrifft, ist, wobei die Soll-Stellung eine Landmarken-Soll-Stellung, welche den Satz von Landmarken betrifft, ist. Der Satz von Landmarken kann beispielsweise basierend auf dem initialen Bilddatensatz berechnet werden. So können in den Bilddaten beispielsweise Landmarken wie die Augen, die Nase, die Ohren und/oder der Mund erfasst und/oder in Bezug auf eine laterale Ausrichtung und/oder eine Links-Rechts-Symmetrie ausgewertet werden.

Eine Ausführungsform sieht vor, dass die Ist-Stellung durch Anwenden eines Algorithmus zur Bewegungsverfolgung auf die Bilddaten berechnet wird, insbesondere indem basierend auf den Bilddaten ein optischer Fluss, ein Block-Matching und/oder eine Bildregistrierung berechnet wird. Die Bilddaten können beispielsweise eine Sequenz von zeitlich aufeinanderfolgenden Bildern umfassen. Auch eine einfache Differenzbildberechnung zur Bestimmung der Lage bzw. von Lageänderungen sind denkbar. Dabei wird vom aktuellen Kamerabild ein Referenzbild subtrahiert, in dem die Position des Untersuchungsobjekts wie gewünscht war. Gibt es Differenzen in den Messwerten der Kamera, so wird ein Hinweis angezeigt.

Eine Ausführungsform sieht vor, dass ein Vektorbild, welches eine Abweichung der Ist-Stellung relativ zu der Soll-Stellung darstellt, berechnet wird, wobei die Einstelldaten für das Einstellsystem basierend auf dem Vektorbild berechnet werden.

Eine Ausführungsform sieht vor, dass basierend auf dem Vektorbild berechnet wird, ob die Abweichung der Ist-Stellung relativ zu der Soll-Stellung außerhalb eines Toleranzbereichs liegt, wobei eine Warnmeldung generiert wird, wenn die Abweichung der Ist-Stellung relativ zu der Soll-Stellung außerhalb des Toleranzbereichs liegt. Die Warnmeldung kann beispielsweise optisch und/oder akustisch ausgegeben werden, insbesondere an medizinisches Personal ausgegeben werden. Abweichungen können dem medizinischen Personal beispielsweise vor der Untersuchung mit einem entsprechenden Hinweis angezeigt werden, etwa, den Kopf des Menschen um eine bestimmte Gradzahl zu drehen.

Eine Ausführungsform sieht vor, dass die Bilddaten optische Bilddaten umfassen, wobei die optischen Bilddaten mittels einer Kamera erfasst werden.

Eine Ausführungsform sieht vor, dass die Bilddaten radiologische Bilddaten umfassen, wobei die radiologischen Bilddaten mittels des medizinischen Bildgebungsgeräts erfasst werden. Die radiologischen Bilddaten können beispielsweise ein Topogramm, insbesondere ein anterior-posterior-Topogramm und/oder ein multi-direktionales Topogramm, umfassen. Das multi-direktionale Topogramm kann beispielsweise ein Rotations-Topogramm sein.

Auf einem lateralen Topogram eines Kopfes ist eine Schieflage des Kopfes oft nicht zu erkennen. Im lateralen Topogramm ist zwar eine grobe automatische Erkennung einer Drehung des Kopfes denkbar. Die eigentlich relevante Position entlang der links/rechts-Achse äußert sich hingegen nur in der projektiven Vergrößerung/Verkleinerung und ist daher ohne Kenntnis der Maße des Bildobjekts nicht bestimmbar.

Eine Ausführungsform sieht vor, dass die laterale Translationsbewegung der Lagerungsstruktur basierend auf der Soll-Stellung als Führungsgröße und der Ist-Stellung als Regelgröße geregelt wird.

Insbesondere kann vorgesehen sein, dass das Erfassen der Bilddaten, das Berechnen der Ist-Stellung, das Berechnen der Einstelldaten für das Einstellsystem und das Einstellen der Position des Untersuchungsobjekts zumindest bis zu einem Zeitpunkt, an dem eine vorgegebene Bedingung, welche eine Abweichung der Ist-Stellung von der Soll-Stellung betrifft, erstmalig erfüllt ist, fortlaufend erfolgt.

Insbesondere kann vorgesehen sein, dass das Erfassen der optischen Bilddaten, das Berechnen der Ist-Stellung, das Berechnen der Einstelldaten für das Einstellsystem und das Einstellen der Position des Untersuchungsobjekts bis zu einem Zeitpunkt, an dem ein Abbruchsignal empfangen wird, fortlaufend erfolgt.

Insbesondere kann die Abweichung der Ist-Stellung von der Soll-Stellung bis zu dem Zeitpunkt, an dem die vorgegebene Bedingung, welche die Abweichung der Ist-Stellung von der Soll-Stellung betrifft, erstmalig erfüllt ist, fortlaufend berechnet werden. Insbesondere kann beispielsweise mittels einer Benutzerschnittstelle angezeigt werden, dass die vorgegebene Bedingung, welche die Abweichung der Ist-Stellung von der Soll-Stellung betrifft, erfüllt ist.

Damit kann die Ist-Stellung fortlaufend überprüft und an die Soll-Stellung angeglichen werden. Die vorgegebene Bedingung, welche die Abweichung der Ist-Stellung von der Soll-Stellung betrifft, kann insbesondere dann erfüllt sein, wenn die Ist-Stellung mit der Soll-Stellung hinreichend übereinstimmt, insbesondere übereinstimmt. Die vorgegebene Bedingung, welche die Abweichung der Ist-Stellung von der Soll-Stellung betrifft, kann ins-besondere dann erfüllt sein, wenn die Abweichung der Ist-Stellung relativ zu der Soll-Stellung innerhalb des Toleranzbereichs liegt.

Insbesondere ist es denkbar, die Position des Untersuchungsobjekts relativ zu der Gantry in Echtzeit zu überwachen und bei Erreichen der gewünschten Ausrichtung ein entsprechendes Signal zu geben. Insbesondere ist es vorteilhaft, die Lage des Patienten und/oder des Kopfes auch nach der optimalen Ausrichtung vor und während der Untersuchung weiter auf Bewegungen hin zu tracken, und das medizinische Personal entsprechend zu informieren.

Die Erfindung betrifft ferner ein Verfahren zum Bereitstellen von Bildgebungsdaten, welche ein Untersuchungsobjekt betreffen, das Verfahren umfassend:
- ein initiales Positionieren des Untersuchungsobjekts in einer Soll-Stellung, welche das Untersuchungsobjekt betrifft, relativ zu einer Gantry eines medizinischen Bildgebungsgeräts, wobei das Untersuchungsobjekt auf einer Lagerungsstruktur eines Untersuchungstisches des medizinischen Bildgebungsgeräts gelagert ist,
- ein Ausführen des erfindungsgemäßen Verfahrens zum Einstellen der Position des Untersuchungsobjekts relativ zu der Gantry,
- eine Akquisition der Bildgebungsdaten, welche das Untersuchungsobjekt betreffen, mittels des medizinischen Bildgebungsgeräts,
- ein Bereitstellen der Bildgebungsdaten, welche das Untersuchungsobjekt betreffen.

Die Erfindung betrifft ferner ein System, aufweisend:
- ein medizinisches Bildgebungsgerät mit einer Gantry und einem Untersuchungstisch, wobei der Untersuchungstisch eine Lagerungsstruktur zum Lagern des Untersuchungsobjekts und ein Einstellsystem aufweist, wobei die Gantry eine Öffnung aufweist, wobei sich die Öffnung derart tunnelförmig entlang einer Systemachse der Gantry erstreckt, dass die Lagerungsstruktur entlang der Systemachse in die Öffnung einführbar ist, wobei mittels des Einstellsystems eine laterale Translationsbewegung der Lagerungsstruktur relativ zu der Systemachse ausgeführt werden kann, wobei die laterale Translationsbewegung horizontal und zu der Systemachse senkrecht ist,
- eine Bilddaten-Erfassungseinheit zum Erfassen von Bilddaten, welche das Untersuchungsobjekt betreffen,
- ein Datenverarbeitungssystem, welches eingerichtet ist, zum
- Berechnen einer Ist-Stellung, welche das Untersuchungsobjekt betrifft, basierend auf den Bilddaten,
- Berechnen, basierend auf der Ist-Stellung und einer Soll-Stellung, welche das Untersuchungsobjekt betrifft, von Einstelldaten für das Einstellsystem, um die Ist-Stellung durch die laterale Translationsbewegung der Lagerungsstruktur relativ zu der Systemachse im Sinne einer Angleichung an die Soll-Stellung zu beeinflussen,
- Einstellen der Position des Untersuchungsobjekts relativ zu der Gantry, indem die laterale Translationsbewegung der Lagerungsstruktur relativ zu der Systemachse mittels des Einstellsystems basierend auf den Einstelldaten ausgeführt wird.

Insbesondere kann vorgesehen sein, dass das System zum Ausführen eines Verfahrens nach einem der offenbarten Aspekte ausgebildet ist.

Die Datenverarbeitungseinheit kann beispielsweise einen Regler bilden, wobei die laterale Translationsbewegung der Lagerungsstruktur basierend auf der Soll-Stellung als Führungsgröße und der Ist-Stellung als Regelgröße mittels des Reglers geregelt wird.

Das medizinische Bildgebungsgerät kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, ins-besondere einem PET-CT-Gerät und einem PET-MR-Gerät, besteht. Das medizinische Bildgebungsgerät kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Eine Ausführungsform sieht vor, dass die Bilddaten-Erfassungseinheit eine Kamera zum Erfassen von optischen Bilddaten, welche das Untersuchungsobjekt betreffen, aufweist, wobei die Bilddaten die optischen Bilddaten umfassen. Die Kamera kann beispielsweise derart relativ zu dem medizinischen Bildgebungsgerät angeordnet sein, dass das Erfassen der optischen Bilddaten zeitlich parallel zu der Akquisition der Bildgebungsdaten erfolgen kann. Die Kamera kann beispielsweise eine 3D-Kamera und/oder eine 2D-Kamera sein. Die Kamera kann beispielsweise an der Gantry und/oder an der Decke eines Untersuchungsraumes befestigt sein.

Eine Ausführungsform sieht vor, dass der Untersuchungstisch einen Sockel aufweist, wobei der Sockel relativ zu der Systemachse ruhend, insbesondere relativ zu der Gantry ruhend, angeordnet ist, wobei das Einstellsystem eine laterale Positioniereinheit aufweist, die dazu eingerichtet ist, die Lagerungsstruktur relativ zu dem Sockel entlang einer lateralen Raumachse zu bewegen, wobei die laterale Raumachse horizontal und zu der Systemachse senkrecht ist. Insbesondere kann vorgesehen sein, dass die laterale Translationsbewegung der Lagerungsstruktur relativ zu der Systemachse mittels des Einstellsystems ausgeführt wird, indem die Lagerungsstruktur mittels der lateralen Positioniereinheit relativ zu dem Sockel entlang der lateralen Raumachse bewegt wird.

Eine Ausführungsform sieht vor, dass das Einstellsystem eine axiale Positioniereinheit aufweist, die dazu eingerichtet ist, die Lagerungsstruktur relativ zu dem Sockel entlang einer axialen Raumachse zu bewegen, wobei die axiale Raumachse horizontal und zu der Systemachse parallel ist. Insbesondere kann vorgesehen sein, dass die Lagerungsstruktur entlang der Systemachse in die Öffnung eingeführt wird, indem die Lagerungsstruktur mittels der axialen Positioniereinheit relativ zu dem Sockel entlang der axialen Raumachse bewegt wird.

Insbesondere kann vorgesehen sein, dass das Einstellsystem eine vertikale Positioniereinheit aufweist, die dazu eingerichtet ist, die Lagerungsstruktur relativ zu dem Sockel entlang einer vertikalen Raumachse zu bewegen, wobei die vertikale Raumachse zu der Systemachse senkrecht ist. Die Lagerungsstruktur kann beispielsweise eine Tischplatte und/oder eine Kopfschale aufweisen. Die Kopfschale kann insbesondere relativ zu der Tischplatte ruhend an der Tischplatte befestigt sein.

Insbesondere kann vorgesehen sein, dass die Systemachse parallel zu einer Längsrichtung der Tischplatte ist. Insbesondere kann basierend auf Kamerabilddaten einer 3D-Kamera die Tischhöhe automatisch eingestellt werden, sodass das Untersuchungsobjekt, hier konkret der Kopf, im Isozentrum der Gantry positioniert wird. Damit wird unterstützt, dass die Dosismodulation den besten Kompromiss aus Bildqualität und Dosis erreicht.

Daten, insbesondere die Bilddaten und/oder der initiale Bilddatensatz, können beispielsweise empfangen werden, indem ein Signal, welches die Daten trägt, empfangen wird und/oder indem die Daten geladen werden, insbesondere aus einem Datenspeicher geladen werden. Daten, insbesondere die Einstelldaten, können beispielsweise bereitgestellt werden, indem ein Signal, welches die Daten trägt, übertragen wird und/oder indem die Daten in einem Datenspeicher abgespeichert werden und/oder indem die Daten auf einem Bildschirm angezeigt werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden.

Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann. Der Ausdruck "basierend auf" kann im Kontext der vorliegenden Anmeldung insbesondere im Sinne des Ausdrucks "unter Verwendung von" verstanden werden.

Im Folgenden werden Merkmale der Erfindung anhand von Beispielen unter Hinweis auf die beigefügten Figuren erläutert. Die Darstellung in den Figuren ist schematisch, stark vereinfacht und nicht zwingend maßstabsgetreu.
Die Fig. 1 zeigt ein System.
Die Fig. 2 zeigt den Untersuchungstisch in einem ersten Betriebszustand.
Die Fig. 3 zeigt den Untersuchungstisch in einem zweiten Betriebszustand.
Die Fig. 4 zeigt ein Ablaufdiagramm eines Verfahrens zum Einstellen einer Position eines Untersuchungsobjekts relativ zu einer Gantry.
Die Fig. 5 zeigt ein Ablaufdiagramm eines Verfahrens zum Bereitstellen von Bildgebungsdaten.

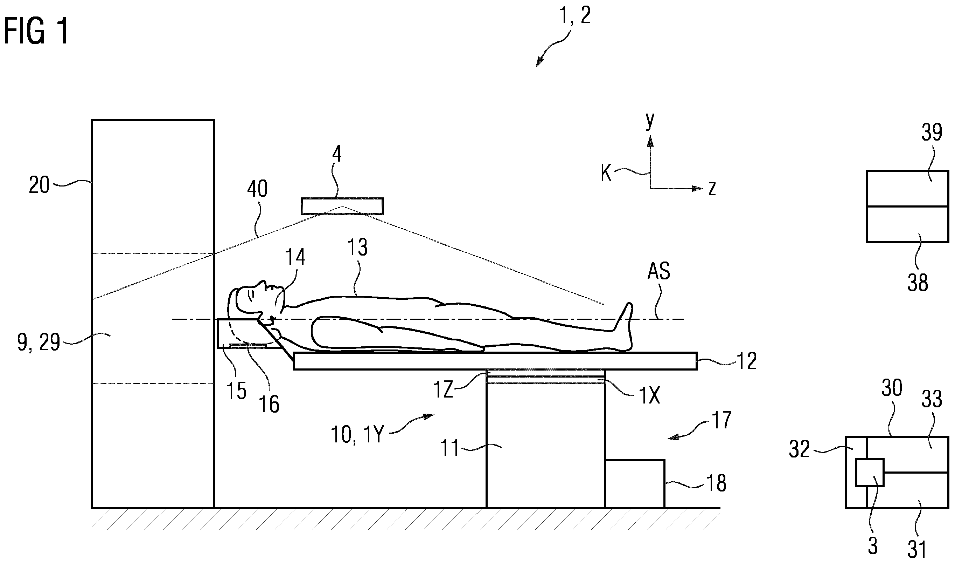

Die Fig. 1 zeigt das System 1, aufweisend:
- ein medizinisches Bildgebungsgerät 2 mit einer Gantry 20 und einem Untersuchungstisch 10, wobei der Untersuchungstisch 10 eine Lagerungsstruktur 12 zum Lagern LK des Untersuchungsobjekts 14 und ein Einstellsystem 17 aufweist, wobei die Gantry 20 eine Öffnung 9 aufweist, wobei sich die Öffnung 9 derart tunnelförmig entlang einer Systemachse SA der Gantry 20 erstreckt, dass die Lagerungsstruktur 12 entlang der Systemachse SA in die Öffnung 9 einführbar ist, wobei mittels des Einstellsystems 17 eine laterale Translationsbewegung der Lagerungsstruktur 12 relativ zu der Systemachse SA ausgeführt werden kann, wobei die laterale Translationsbewegung horizontal und zu der Systemachse SA senkrecht ist,
- eine Bilddaten-Erfassungseinheit zum Erfassen EO von Bilddaten, welche das Untersuchungsobjekt 14 betreffen,
- ein Datenverarbeitungssystem 3, welches eingerichtet ist, zum
- Berechnen BI einer Ist-Stellung, welche das Untersuchungsobjekt 14 betrifft, basierend auf den Bilddaten,
- Berechnen BN, basierend auf der Ist-Stellung und einer Soll-Stellung, welche das Untersuchungsobjekt 14 betrifft, von Einstelldaten für das Einstellsystem 17, um die Ist-Stellung durch die laterale Translationsbewegung der Lagerungsstruktur 12 relativ zu der Systemachse SA im Sinne einer Angleichung an die Soll-Stellung zu beeinflussen,
- Einstellen NK der Position des Untersuchungsobjekts 14 relativ zu der Gantry 20, indem die laterale Translationsbewegung der Lagerungsstruktur 12 relativ zu der Systemachse SA mittels des Einstellsystems 17 basierend auf den Einstelldaten ausgeführt wird.

Das gezeigte Beispiel sieht vor, dass die Bilddaten-Erfassungseinheit eine Kamera 4 zum Erfassen von optischen Bilddaten, welche das Untersuchungsobjekt 14 betreffen, aufweist, wobei die Bilddaten die optischen Bilddaten umfassen.

In der tunnelförmigen Öffnung 9 befindet sich der Akquisitionsbereich 29 des medizinischen Bildgebungsgeräts 2, in dem das medizinische Bildgebungsgerät 2 mit dem Untersuchungsobjekt 14 wechselwirken kann, um Bildgebungsdaten von dem Untersuchungsobjekt 14 zu akquirieren. Der Akquisitionsbereich 29 befindet sich im Sichtfeld 40 der Kamera 4. Die Kamera 4 ist derart relativ zu dem medizinischen Bildgebungsgerät 2 angeordnet, dass das Erfassen der optischen Bilddaten zeitlich parallel zu der Akquisition der Bildgebungsdaten erfolgen kann. Die Raumachsen x, y und z bilden das Koordinatensystem K. Die Lagerungsstruktur 12 weist eine Tischplatte und die Kopfschale 15 mit der Kopfunterlage 16 auf. Das Untersuchungsobjekt 14 ist der Kopf des Patienten 13.

Das System 1 weist ferner einen Computer 30 zur Steuerung des medizinischen Bildgebungsgeräts 2, der Kamera 4 und des Einstellsystems 17 auf. Der Computer 30 weist einen Prozessor 33, einen Speicher 31 und eine Datenübertragungsschnittstelle 32 auf und bildet das Datenverarbeitungssystem 3. Das System 1 weist eine Benutzerschnittstelle, beispielsweise in Form eines berührungsempfindlichen Bildschirms, auf. Die Benutzerschnittstelle weist ein Eingabefeld 38, beispielsweise zum manuellen Festlegen der Soll-Stellung, und ein Ausgabefeld 39, beispielsweise zur optischen Ausgabe der Einstelldaten und/oder der Warnmeldung auf.

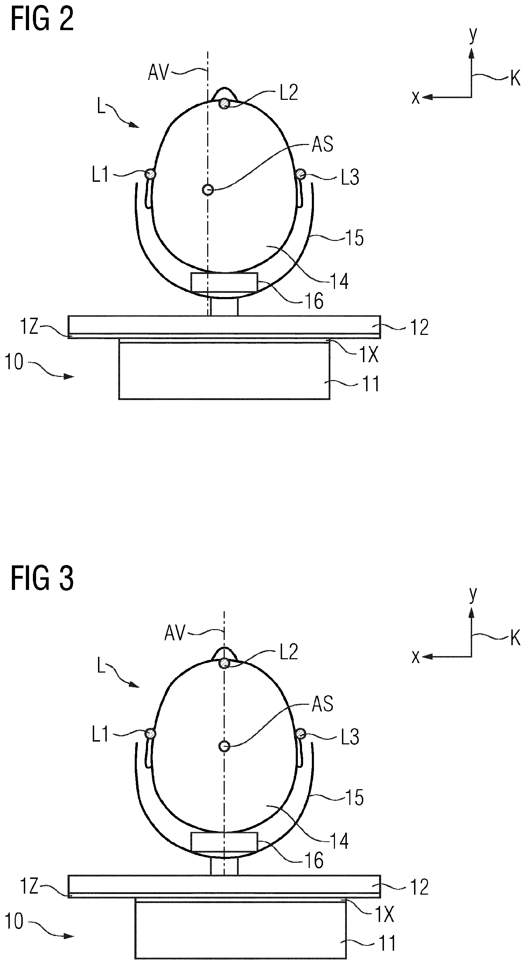

Die Fig. 2 zeigt den Untersuchungstisch 10 in einem ersten Betriebszustand. Der Satz von Landmarken L umfasst die drei Landmarken L1, L2 und L3. Die Systemachse AS ist horizontal und liegt in der vertikalen Ebene AV.

Das gezeigte Beispiel sieht vor, dass der Untersuchungstisch 10 einen Sockel 11 aufweist, wobei der Sockel 11 relativ zu der Systemachse SA ruhend angeordnet ist, wobei das Einstellsystem 17 eine laterale Positioniereinheit 1X aufweist, die dazu eingerichtet ist, die Lagerungsstruktur 12 relativ zu dem Sockel 11 entlang einer lateralen Raumachse x zu bewegen, wobei die laterale Raumachse x horizontal und zu der Systemachse SA senkrecht ist. Das gezeigte Beispiel sieht vor, dass die laterale Translationsbewegung der Lagerungsstruktur 12 relativ zu der Systemachse SA mittels des Einstellsystems 17 ausgeführt wird, indem die Lagerungsstruktur 12 mittels der lateralen Positioniereinheit 1X relativ zu dem Sockel 11 entlang der lateralen Raumachse x bewegt wird.

Das gezeigte Beispiel sieht vor, dass das Einstellsystem 17 eine axiale Positioniereinheit 1Z aufweist, die dazu eingerichtet ist, die Lagerungsstruktur 12 relativ zu dem Sockel 11 entlang einer axialen Raumachse z zu bewegen, wobei die axiale Raumachse z horizontal und zu der Systemachse SA parallel ist. Das gezeigte Beispiel sieht vor, dass die Lagerungsstruktur 12 entlang der Systemachse SA in die Öffnung 9 eingeführt wird, indem die Lagerungsstruktur 12 mittels der axialen Positioniereinheit 1Z relativ zu dem Sockel 11 entlang der axialen Raumachse z bewegt wird. Das gezeigte Beispiel sieht vor, dass das Einstellsystem 17 eine vertikale Positioniereinheit 1Y aufweist, die dazu eingerichtet ist, die Lagerungsstruktur 12 relativ zu dem Sockel 11 entlang einer vertikalen Raumachse y zu bewegen, wobei die vertikale Raumachse z zu der Systemachse SA senkrecht ist.

Die Fig. 3 zeigt den Untersuchungstisch 10 in einem zweiten Betriebszustand, in dem die Ist-Stellung mit der Soll-Stellung übereinstimmt.

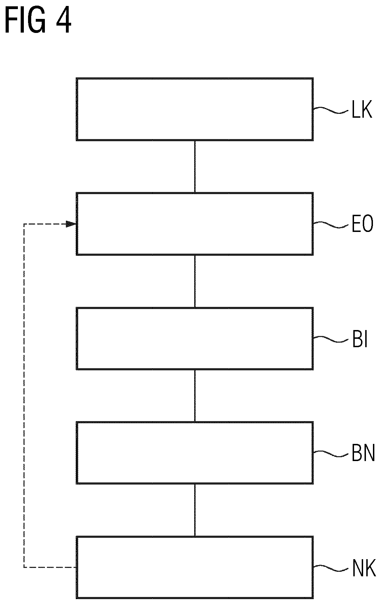

Die Fig. 4 zeigt ein Ablaufdiagramm eines Verfahrens zum Einstellen einer Position eines Untersuchungsobjekts 14 relativ zu einer Gantry 20 eines medizinischen Bildgebungsgeräts 2, das Verfahren umfassend:
- ein Lagern LK des Untersuchungsobjekts 14 auf einer Lagerungsstruktur 12 eines Untersuchungstisches 10 des medizinischen Bildgebungsgeräts 2, wobei das medizinische Bildgebungsgerät 2 die Gantry 20 mit einer Öffnung 9 aufweist, wobei sich die Öffnung 9 derart tunnelförmig entlang einer Systemachse SA der Gantry 20 erstreckt, dass die Lagerungsstruktur 12 entlang der Systemachse SA in die Öffnung 9 einführbar ist, wobei der Untersuchungstisch 10 ein Einstellsystem 17 aufweist, wobei mittels des Einstellsystems 17 eine laterale Translationsbewegung der Lagerungsstruktur 12 relativ zu der Systemachse SA ausgeführt werden kann, wobei die laterale Translationsbewegung horizontal und zu der Systemachse SA senkrecht ist,
- ein Erfassen EO von Bilddaten, welche das Untersuchungsobjekt 14 betreffen,
- ein Berechnen BI einer Ist-Stellung, welche das Untersuchungsobjekt 14 betrifft, basierend auf den Bilddaten,
- ein Berechnen BN, basierend auf der Ist-Stellung und einer Soll-Stellung, welche das Untersuchungsobjekt 14 betrifft, von Einstelldaten für das Einstellsystem 17, um die Ist-Stellung durch die laterale Translationsbewegung der Lagerungsstruktur 12 relativ zu der Systemachse SA im Sinne einer Angleichung an die Soll-Stellung zu beeinflussen,
- ein Einstellen NK der Position des Untersuchungsobjekts 14 relativ zu der Gantry 20, indem die laterale Translationsbewegung der Lagerungsstruktur 12 relativ zu der Systemachse SA mittels des Einstellsystems 17 basierend auf den Einstelldaten ausgeführt wird.

Das gezeigte Beispiel sieht vor, dass ein initialer Bilddatensatz, welcher das Untersuchungsobjekt 14 betrifft, empfangen wird, wobei die Soll-Stellung basierend auf dem initialen Bilddatensatz berechnet wird. Das gezeigte Beispiel sieht vor, dass eine Soll-Pose des Untersuchungsobjekts 14 basierend auf dem initialen Bilddatensatz berechnet wird, wobei die Soll-Stellung basierend auf der Soll-Pose des Untersuchungsobjekts 14 berechnet wird. Das gezeigte Beispiel sieht vor, dass die Ist-Stellung eine Landmarken-Ist-Stellung, welche einen Satz von Landmarken L an dem Untersuchungsobjekt 14 betrifft, ist, wobei die Soll-Stellung eine Landmarken-Soll-Stellung, welche den Satz von Landmarken L betrifft, ist.

Das gezeigte Beispiel sieht vor, dass die Ist-Stellung durch Anwenden eines Algorithmus zur Bewegungsverfolgung auf die Bilddaten berechnet wird. Das gezeigte Beispiel sieht vor, dass ein Vektorbild, welches eine Abweichung der Ist-Stellung relativ zu der Soll-Stellung darstellt, berechnet wird, wobei die Einstelldaten für das Einstellsystem basierend auf dem Vektorbild berechnet werden.

Das gezeigte Beispiel sieht vor, dass basierend auf dem Vektorbild berechnet wird, ob die Abweichung der Ist-Stellung relativ zu der Soll-Stellung außerhalb eines Toleranzbereichs liegt, wobei eine Warnmeldung generiert wird, wenn die Abweichung der Ist-Stellung relativ zu der Soll-Stellung außerhalb des Toleranzbereichs liegt. Das gezeigte Beispiel sieht vor, dass die Bilddaten optische Bilddaten umfassen, wobei die optischen Bilddaten mittels einer Kamera 4 erfasst werden. Das gezeigte Beispiel sieht vor, dass die Bilddaten radiologische Bilddaten umfassen, wobei die radiologischen Bilddaten mittels des medizinischen Bildgebungsgeräts 2 erfasst werden.

Das gezeigte Beispiel sieht vor, dass die laterale Translationsbewegung der Lagerungsstruktur 12 basierend auf der Soll-Stellung als Führungsgröße und der Ist-Stellung als Regelgröße geregelt wird. Die gestrichelte Linie veranschaulicht das fortlaufende Regeln der lateralen Translationsbewegung der Lagerungsstruktur 12 basierend auf der Soll-Stellung als Führungsgröße und der Ist-Stellung als Regelgröße.

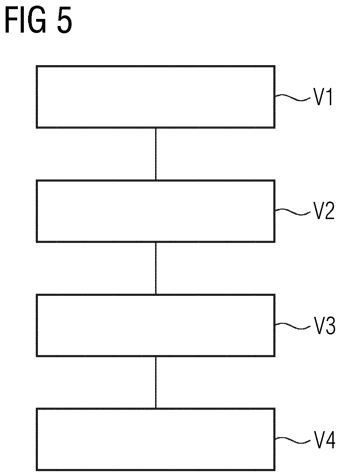

Die Fig. 5 zeigt ein Ablaufdiagramm eines Verfahrens zum Bereitstellen von Bildgebungsdaten, welche ein Untersuchungsobjekt 14 betreffen, das Verfahren umfassend:
- ein initiales Positionieren V1 des Untersuchungsobjekts 14 in einer Soll-Stellung, welche das Untersuchungsobjekt 14 betrifft, relativ zu einer Gantry 20 eines medizinischen Bildgebungsgeräts 2, wobei das Untersuchungsobjekt 14 auf einer Lagerungsstruktur 12 eines Untersuchungstisches 10 des medizinischen Bildgebungsgeräts 2 gelagert ist,
- ein Ausführen V2 des in der Fig. 4 gezeigten Verfahrens zum Einstellen der Position des Untersuchungsobjekts 14 relativ zu der Gantry 20,
- eine Akquisition V3 der Bildgebungsdaten, welche das Untersuchungsobjekt 14 betreffen, mittels des medizinischen Bildgebungsgeräts 2,
- ein Bereitstellen V4 der Bildgebungsdaten, welche das Untersuchungsobjekt 14 betreffen.

## Patentansprüche

1. Verfahren zum Einstellen einer Position eines Untersuchungsobjekts (14) relativ zu einer Gantry (20) eines medizinischen Bildgebungsgeräts (2), das Verfahren umfassend:
- ein Lagern (LK) des Untersuchungsobjekts (14) auf einer Lagerungsstruktur (12) eines Untersuchungstisches (10) des medizinischen Bildgebungsgeräts (2), wobei das medizinische Bildgebungsgerät (2) die Gantry (20) mit einer Öffnung (9) aufweist, wobei sich die Öffnung (9) derart tunnelförmig entlang einer Systemachse (SA) der Gantry (20) erstreckt, dass die Lagerungsstruktur (12) entlang der Systemachse (SA) in die Öffnung (9) einführbar ist, wobei der Untersuchungstisch (10) ein Einstellsystem (17) aufweist, wobei mittels des Einstellsystems (17) eine laterale Translationsbewegung der Lagerungsstruktur (12) relativ zu der Systemachse (SA) ausgeführt werden kann, wobei die laterale Translationsbewegung horizontal und zu der Systemachse (SA) senkrecht ist,
- ein Erfassen (EO) von Bilddaten, welche das Untersuchungsobjekt (14) betreffen,
- ein Berechnen (BI) einer Ist-Stellung, welche das Untersuchungsobjekt (14) betrifft, basierend auf den Bilddaten,
- ein Berechnen (BN), basierend auf der Ist-Stellung und einer Soll-Stellung, welche das Untersuchungsobjekt (14) betrifft, von Einstelldaten für das Einstellsystem (17), um die Ist-Stellung durch die laterale Translationsbewegung der Lagerungsstruktur (12) relativ zu der Systemachse (SA) im Sinne einer Angleichung an die Soll-Stellung zu beeinflussen,
- ein Einstellen (NK) der Position des Untersuchungsobjekts (14) relativ zu der Gantry (20), indem die laterale Translationsbewegung der Lagerungsstruktur (12) relativ zu der Systemachse (SA) mittels des Einstellsystems (17) basierend auf den Einstelldaten ausgeführt wird.

2. Verfahren nach Anspruch 1,
- wobei ein initialer Bilddatensatz, welcher das Untersuchungsobjekt (14) betrifft, empfangen wird,
- wobei die Soll-Stellung basierend auf dem initialen Bilddatensatz berechnet wird.

3. Verfahren nach Anspruch 2,
- wobei eine Soll-Pose des Untersuchungsobjekts (14) basierend auf dem initialen Bilddatensatz berechnet wird,
- wobei die Soll-Stellung basierend auf der Soll-Pose des Untersuchungsobjekts (14) berechnet wird.

4. Verfahren nach einem der Ansprüche 1 bis 3,
- wobei die Ist-Stellung eine Landmarken-Ist-Stellung, welche einen Satz von Landmarken (L) an dem Untersuchungsobjekt (14) betrifft, ist,
- wobei die Soll-Stellung eine Landmarken-Soll-Stellung, welche den Satz von Landmarken (L) betrifft, ist.

5. Verfahren nach einem der Ansprüche 1 bis 4,
- wobei die Ist-Stellung durch Anwenden eines Algorithmus zur Bewegungsverfolgung auf die Bilddaten berechnet wird.

6. Verfahren nach einem der Ansprüche 1 bis 5,
- wobei ein Vektorbild, welches eine Abweichung der Ist-Stellung relativ zu der Soll-Stellung darstellt, berechnet wird,
- wobei die Einstelldaten für das Einstellsystem basierend auf dem Vektorbild berechnet werden.

7. Verfahren nach Anspruch 6,
- wobei basierend auf dem Vektorbild berechnet wird, ob die Abweichung der Ist-Stellung relativ zu der Soll-Stellung außerhalb eines Toleranzbereichs liegt,
- wobei eine Warnmeldung generiert wird, wenn die Abweichung der Ist-Stellung relativ zu der Soll-Stellung außerhalb des Toleranzbereichs liegt.

8. Verfahren nach einem der Ansprüche 1 bis 7,
- wobei die Bilddaten optische Bilddaten umfassen,
- wobei die optischen Bilddaten mittels einer Kamera (4) erfasst werden.

9. Verfahren nach einem der Ansprüche 1 bis 8,
- wobei die Bilddaten radiologische Bilddaten umfassen,
- wobei die radiologischen Bilddaten mittels des medizinischen Bildgebungsgeräts (2) erfasst werden.

10. Verfahren nach einem der Ansprüche 1 bis 9,
- wobei die laterale Translationsbewegung der Lagerungsstruktur (12) basierend auf der Soll-Stellung als Führungsgröße und der Ist-Stellung als Regelgröße geregelt wird.

11. Verfahren zum Bereitstellen von Bildgebungsdaten, welche ein Untersuchungsobjekt (14) betreffen, das Verfahren umfassend:
- ein initiales Positionieren (V1) des Untersuchungsobjekts (14) in einer Soll-Stellung, welche das Untersuchungsobjekt (14) betrifft, relativ zu einer Gantry (20) eines medizinischen Bildgebungsgeräts (2), wobei das Untersuchungsobjekt (14) auf einer Lagerungsstruktur (12) eines Untersuchungstisches (10) des medizinischen Bildgebungsgeräts (2) gelagert ist,
- ein Ausführen (V2) des Verfahrens nach einem der Ansprüche 1 bis 10 zum Einstellen der Position des Untersuchungsobjekts (14) relativ zu der Gantry (20),
- eine Akquisition (V3) der Bildgebungsdaten, welche das Untersuchungsobjekt (14) betreffen, mittels des medizinischen Bildgebungsgeräts (2),
- ein Bereitstellen (V4) der Bildgebungsdaten, welche das Untersuchungsobjekt (14) betreffen.

12. System (1), aufweisend:
- ein medizinisches Bildgebungsgerät (2) mit einer Gantry (20) und einem Untersuchungstisch (10), wobei der Untersuchungstisch (10) eine Lagerungsstruktur (12) zum Lagern (LK) des Untersuchungsobjekts (14) und ein Einstellsystem (17) aufweist, wobei die Gantry (20) eine Öffnung (9) aufweist, wobei sich die Öffnung (9) derart tunnelförmig entlang einer Systemachse (SA) der Gantry (20) erstreckt, dass die Lagerungsstruktur (12) entlang der Systemachse (SA) in die Öffnung (9) einführbar ist, wobei mittels des Einstellsystems (17) eine laterale Translationsbewegung der Lagerungsstruktur (12) relativ zu der Systemachse (SA) ausgeführt werden kann, wobei die laterale Translationsbewegung horizontal und zu der Systemachse (SA) senkrecht ist,
- eine Bilddaten-Erfassungseinheit zum Erfassen (EO) von Bilddaten, welche das Untersuchungsobjekt (14) betreffen,
- ein Datenverarbeitungssystem (3), welches eingerichtet ist zum
- Berechnen (BI) einer Ist-Stellung, welche das Untersuchungsobjekt (14) betrifft, basierend auf den Bilddaten,
- Berechnen (BN), basierend auf der Ist-Stellung und einer Soll-Stellung, welche das Untersuchungsobjekt (14) betrifft, von Einstelldaten für das Einstellsystem (17), um die Ist-Stellung durch die laterale Translationsbewegung der Lagerungsstruktur (12) relativ zu der Systemachse (SA) im Sinne einer Angleichung an die Soll-Stellung zu beeinflussen,
- Einstellen (NK) der Position des Untersuchungsobjekts (14) relativ zu der Gantry (20), indem die laterale Translationsbewegung der Lagerungsstruktur (12) relativ zu der Systemachse (SA) mittels des Einstellsystems (17) basierend auf den Einstelldaten ausgeführt wird.

13. System (1) nach Anspruch 12,
- wobei die Bilddaten-Erfassungseinheit eine Kamera (4) zum Erfassen von optischen Bilddaten, welche das Untersuchungsobjekt (14) betreffen, aufweist,
- wobei die Bilddaten die optischen Bilddaten umfassen.

14. System (1) nach Anspruch 12 oder 13,
- wobei der Untersuchungstisch (10) einen Sockel (11) aufweist, wobei der Sockel (11) relativ zu der Systemachse (SA) ruhend angeordnet ist,
- wobei das Einstellsystem (17) eine laterale Positioniereinheit (1X) aufweist, die dazu eingerichtet ist, die Lagerungsstruktur (12) relativ zu dem Sockel (11) entlang einer lateralen Raumachse (x) zu bewegen, wobei die laterale Raumachse (x) horizontal und zu der Systemachse (SA) senkrecht ist.

15. System (1) nach Anspruch 14,
- wobei das Einstellsystem (17) eine axiale Positioniereinheit (1Z) aufweist, die dazu eingerichtet ist, die Lagerungsstruktur (12) relativ zu dem Sockel (11) entlang einer axialen Raumachse (z) zu bewegen, wobei die axiale Raumachse (z) horizontal und zu der Systemachse (SA) parallel ist.
